# EUROPEAN PATENT APPLICATION

(11) **EP 1 241 281 A1**
(43) Date of publication of application: **18.09.2002**
(21) Application number: 02251833.6
(22) Date of filing: 14.03.2002
(51) Int. Cl.: C25D 5/10, C25D 5/12, C23C 28/02

(54) **Tin plating**

(30) Priority: 16.03.2001 US 276891 P
(71) Applicant: Shipley Co. L.L.C., Marlborough, MA 01752 (US)
(72) Inventor: Heber, Jochen, 6010 Kriens (CH); Egli, Andr-, 8712 Staefa (CH); Toben, Michael P., Smithtown, New York 11787 (US); Schwager, Felix, 6045 Meggen (CH)
(74) Representative: Kent, Venetia Katherine

(57) **Abstract**

Disclosed is a method of reducing whisker formation in tin films by the use of a thin metal undercoat. Also disclosed are structures having tin films with substantially reduced whisker formation.

## Description

### Background of the Invention

In general, the present invention relates to the filed to tin plating. In particular, the present invention relates to the reduction of whisker formation in a tin film.

Tin layers are typically used in the electronics industry to provide good solderability of components. For example, tin layers may be deposited on a copper lead frame to provide a solderable finish. Unfortunately, tin layers are subject to whisker formation. "Whiskers" are hair-like single crystals that grow from the surface of the tin layer. Tin whiskers may range in diameter from 6 nanometers to 6 microns and reach lengths of several millimeters. Such whiskers create shorts and introduce noise into electronic circuitry, thus creating a reliability problem in electronic devices.

It is believed that tin whiskers form as a result of stresses in the tin or tin alloy layer, although bulk diffusion of tin is also believed to be involved. A number of stress-causing factors have been postulated, including lattice stresses due to the presence of impurity atoms in the tin layer; residual stresses due to tin plating conditions; stresses due to mechanical loading or working of the tin layer; stresses due to interaction with adjacent layers, such as intermetallic compound formation, differences in thermal expansion and the like; among others. See, for example, Ewell et al., *Tin Whiskers and Passive Components: A Review of the Concerns,* 18^{th} Capacitor and Resistor Technology Symposium, pp 222-228, March, 1998.

One approach to solving the tin whisker problem has been the use of an underplate or underlayer between the substrate and the tin layer. Such underlayer provides a barrier between the substrate, such as copper, and the tin layer. Such barrier is believed to retard interaction between the substrate metal and the tin layer, thus reducing the formation of intermetallic compounds and thus reduce the formation of tin whiskers. It is believed that such barrier layers need to be of a certain minimum thickness to prevent whisker formation, with the minimum thickness being dependent upon the particular metal or alloy used as the barrier material. For example, Hada et al., *Study of Tin Whiskers on Electromagnetic Relay Parts*, Proceedings of the 25^{th} Annual Relay Conference, 1976, pp 9-1 to 9-15, discloses nickel layers having a thickness of at least 2 microns as a suitable underplate for tin layers. Hada et al. disclose that when a 2 to 5 micron nickel layer is used an underlayer, the deposited tin layers show no signs of whisker formation. Even though such 2 micron thick nickel layers are known, conventional chip capacitors contain a nickel underlayer of 5 to 6 microns. See, for example, Kuhl et al., *Assuring Whisker-free Components*, SMT Magazine, pp 48-49, June, 1995.

In many electronics applications, such as semiconductor lead frame manufacture, ductility of a metal deposit is important. For example, after the outer leads of a lead frame package are plated with tin or tin alloy to assist in solderability, the leads are bent by forming to provide the correct footprint for proper attachment of the component package to a printed circuit board. This forming operation bends the leads 90 degrees or more, creating significant strain on the plated lead. If the electrodeposited finishes on the lead are not sufficiently ductile to withstand the forming operations, the deposit will crack. Should such deposit crack, the copper substrate can be exposed leading to solderability problems. Nickel is not as ductile as tin or tin alloys and thus crack formation in the nickel underlayer is a problem.

Another approach to reducing tin whisker formation has been to use relatively thick tin layers, such as about 10 microns. However, such thick layers are not always practical or they may be too thick for current microelectronic applications.

U.S. Patent No. 5,750,017 discloses a method of depositing tin or tin alloy onto a metal substrate using pulse plating conditions. Such plating method provides a tin layer wherein the grain size of the tin is from 2 to 8 microns. Such grain size is believed to reduce tin whisker formation. Typically, the resulting tin layers have a thickness of 3 to 6 microns.

There is thus a need for methods of providing tin and tin alloy layers, particularly thin layers, having reduced tin whisker formation and with reduced deposit cracks.

### Summary of the Invention

It has been surprisingly found that the thin metal underlayers including nickel or cobalt of the present invention greatly reduce whisker formation in tin films while also greatly reducing cracking of the underlayers.

In one aspect, the present invention provides a method of reducing whisker formation including the step of first depositing a metal underlayer including nickel, nickel alloys, cobalt or cobalt alloys; and then depositing a tin or tin alloy layer on the metal underlayer; wherein the metal underlayer has a thickness of up to 1 µm.

In a second aspect, the present invention provides a method of depositing a tin or tin alloy layer on a substrate including the steps of depositing a metal underlayer including nickel, nickel alloys, cobalt or cobalt alloys on a substrate; and then depositing a tin or tin alloy layer on the metal underlayer; wherein the metal underlayer has a thickness of up to 1 µm.

In a third aspect, the present invention provides a substrate including a metal underlayer including nickel, nickel alloys, cobalt or cobalt alloys disposed on the substrate; and a tin or tin alloy layer disposed on the metal underlayer; wherein the metal underlayer has a thickness of up to 1 µm.

In a fourth aspect, the present invention provides an electronic device including a metal underlayer including nickel, nickel alloys, cobalt or cobalt alloys disposed on the device; and a tin or tin alloy layer disposed on the metal underlayer; wherein the metal underlayer has a thickness of up to 1 µm.

### Detailed Description of the Invention

As used throughout this specification, the following abbreviations shall have the following meanings, unless the context clearly indicates otherwise: ° C = degrees Centigrade; g = gram; L = liter; mL = milliliter; A = amperes; dm = decimeter; and µm = micron = micrometer.

The terms "depositing" and "plating" are used interchangeably throughout this specification. "Underplate" and "underlayer" are used interchangeably throughout this specification. The term "underlayer," as used throughout this specification, refers to a metal layer disposed between a substrate and a tin or tin alloy layer. The terms "printed wiring board" and "printed circuit board" are used interchangeably throughout this specification. All amounts are percent by weight and all ratios are by weight, unless otherwise noted. All numerical ranges are inclusive and combinable in any order, except where it is obvious that such numerical ranges are constrained to add up to 100%.

The present invention provides a method of reducing whisker formation including the step of first depositing a metal underlayer including nickel, nickel alloys, cobalt or cobalt alloys; and then depositing a tin or tin alloy layer on the metal underlayer; wherein the metal underlayer has a thickness of up to 1 µm. Any nickel and cobalt alloys may be used in the present invention. By "nickel alloy" is meant any metal containing nickel and one or more different alloying elements, and thus includes binary alloys, ternary alloys and the like. By "cobalt alloy" is meant any metal containing cobalt and one or more different alloying elements, and thus includes binary alloys, ternary alloys and the like. Suitable alloying elements include, but are not limited to, tungsten, nickel, cobalt, phosphorus, and the like. The amount of nickel in a nickel alloy and cobalt in a cobalt alloy varies over a wide range and is well within the ability of one skilled in the art. Particularly suitable underlayers include nickel, cobalt, nickel-cobalt, nickel-phosphorus or nickel-tungsten. Particularly suitable nickel-phosphorus alloys include those having from 2 to 13 % phosphorus. Such nickel-phosphorus alloys may be deposited either electrolessly or electrolytically and are well within the ability of one skilled in the art.

The underlayers of the present invention may be deposited by a variety of methods. Suitable methods include, but are not limited to, electroless plating, electrolytic plating, immersion plating or vapor deposition such as physical vapor deposition or chemical vapor deposition. It is preferred that the underlayer is deposited by electroless plating or electrolytic plating, and more preferably by electrolytic plating. Such electrolytic deposition may be by either direct current ("DC") or by pulse plating. The choice of the deposit will depend upon the particular substrate, and the layer to be deposited. Such choice is well within the ability of one skilled in the art.

A wide variety of electrolytic nickel baths may be used. Such nickel baths typically contain one or more soluble sources of nickel compounds such as nickel halides such as nickel chloride, nickel sulfate, nickel sulfamate, nickel fluoborate and mixtures thereof. Such nickel compounds are typically employed in concentrations sufficient to provide nickel in the electroplating solutions in concentrations ranging from about 10 to 250 g/L. It is preferred that the nickel electroplating bath contains nickel chloride and nickel sulfamate. It is further preferred that the amount of nickel chloride in the bath is from 8 to 15 g/L and the amount of nickel as nickel sulfamate is from 80 to 150 g/L.

Suitable nickel plating baths typically contain one or more acids, such as boric acid, phosphoric acid, phosphorus acid or mixtures thereof. Exemplary boric acid containing nickel electroplating baths contain from 30 to 60 g/L of boric acid, and preferably about 45 g/L. Typically, the pH of such baths is from about 3.0 to about 5.0, and preferably is about 4.0. The operating temperature of such nickel electroplating baths may range from about 40° to about 70° C, and is preferably from 50° to 65° C. The average cathode current density may range from about 0.5 to 12 amperes per square decimeter with 3 to 6 amperes per square decimeter providing an optimum range.

In nickel alloy plating baths, one or more other alloying metal compounds are used. Such alloying metal compounds are any which provide the metal to the electrolyte composition in a soluble form. Thus, the metal compounds include, but are not limited to, salts, such as metal halides, metal sulfates, metal alkane sulfonate such as metal methane sulfonate, metal aryl sulfonate such as metal phenyl sulfonate and metal toluene sulfonate, metal alkanol sulfonate, and the like. The choice of other metal compound and the amount of such other metal compound present in the electrolyte composition depends upon the tin-alloy to be deposited, and is well known to those skilled in the art.

It will be appreciated by those skilled in the art that one or more other additives may be used the nickel electroplating baths, such as grain refiners, wetting agents, brightening agents and the like. Mixtures of additives may also be used in the present invention. Such additives are useful in enhancing the efficiency and/or quality of the plating

The underlayer of the present invention is typically up to 1 µm thick. Preferably, the underlayer is up to 0.95 µm thick, more preferably up to 0.9 µm thick, still more preferably up to 0.8 µm, even more preferably up to 0.75 µm, still even more preferably up to 0.5 µm. The minimum thickness of the underlayer is that necessary to provide a substantially continuous metal layer over the substrate surface, such that the tin or tin alloy layer does not intimately contact the substrate surface. A suitable minimum thicknesses of the present underlayer is 0.05 µm. In general, suitable ranges of thickness of the underlayer are from 0.05 to 1 µm, preferably from 0.05 to 0.95 µm, more preferably from 0.1 to 0.75 µm and still more preferably from 0,1 to 0.5 µm. Particularly suitable underlayer thicknesses include ≥0.1 µm and ≥ 0.25 µm.

A wide variety of tin alloys may be deposited on the underlayer according to the present invention. "Tin alloy" refers to any metal containing tin and one or more other alloying elements, and thus includes binary alloys, ternary alloys and the like. Suitable alloying elements include, but are not limited to, bismuth, copper, silver, lead, antimony or zinc. A particularly suitable tin alloy is tin-copper. Such tin-copper alloys may contain from 0.5 to 10% copper, the balance being tin. Such tin-copper alloys may optionally contain small amounts of other alloying elements. The concentrations of the one or more alloying elements may vary over a wide range. The range of such alloying elements is well within the ability of one skilled in the art. The thickness of the tin or tin alloy layers may vary across a wide range, depending upon the particular application. For example, when a tin or tin alloy layer is deposited on a lead frame, it is typically from 1 to 10 microns thick.

The tin or tin alloy layer may be deposited by a variety of means. Preferably, the tin or tin alloy is deposited electrolytically. Such electrolytic deposition may be by either direct current ("DC") or by pulse plating. The choice of the deposit will depend upon the particular substrate, and the layer to be deposited. Such choice is well within the ability of one skilled in the art.

Suitable electrolytic tin or tin alloy plating baths may be acidic or alkaline. An exemplary acidic tin electroplating bath contains one or more solution soluble tin compounds, one or more acidic electrolytes, and optionally one or more additives. Suitable tin compounds include, but are not limited to salts, such as tin halides, tin sulfates, tin alkane sulfonate such as tin methane sulfonate, tin aryl sulfonate such as tin phenyl sulfonate and tin toluene sulfonate, till alkanol sulfonate, and the like. It is preferred that the tin compound is tin sulfate, tin chloride, tin alkane sulfonate or tin aryl sulfonate, and more preferably tin sulfate or tin methane sulfonate. The amount of tin compound useful in the electrolyte compositions of the present invention is any amount that provides a tin content typically in the range of 5 to 150 g/L, and preferably 10 to 70 g/L. Mixtures of tin compounds may also be used advantageously in the present invention, provided that the total amount of tin is in the range of from 5 to 150 g/L.

Any acidic electrolyte that is solution soluble and does not otherwise adversely affect the electrolyte composition may be used advantageously in the present invention. Suitable acidic electrolytes include, but are not limited to alkane sulfonic acids, such as methane sulfonic acid, ethane sulfonic acid and propane sulfonic acid, aryl sulfonic acids such as phenyl sulfonic acid or toluene sulfonic acid, sulfuric acid, sulfamic acid, hydrochloric acid, hydrobromic acid, fluoroboric acid and mixtures thereof. Typically, the amount of acidic electrolyte is in the range of 10 to 400 g/L, and preferably 50 to 200 g/L. It is preferred that when the tin compound is a halide that the acidic electrolyte is the corresponding acid. For example, when tin chloride is used in the present invention, it is preferred that the acidic electrolyte is hydrochloric acid.

In tin alloy plating baths, one or more other alloying metal compounds are used. Suitable other metals include, but are not limited to, lead, nickel, copper, bismuth, zinc, silver, antimony, indium and the like. A particularly suitable tin alloy is tin-copper. The other metal compounds useful in the present invention are any which provide the metal to the electrolyte composition in a soluble form. Thus, the metal compounds include, but are not limited to, salts, such as metal halides, metal sulfates, metal alkane sulfonate such as metal methane sulfonate, metal aryl sulfonate such as metal phenyl sulfonate and metal toluene sulfonate, metal alkanol sulfonate, and the like. The choice of other metal compound and the amount of such other metal compound present in the electrolyte composition depends upon the tin-alloy to be deposited, and is well known to those skilled in the art.

It will be appreciated by those skilled in the art that one or more other additives may be used in the tin or tin alloy electroplating baths, such as reducing agents, grain refiners such as hydroxy aromatic compounds and other wetting agents, brightening agents and the like. Mixtures of additives may also be used in the present invention. Such additives are useful in enhancing the efficiency and/or quality of the plating

Reducing agents may be added to the electrolyte composition of the present invention to assist in keeping the tin in a soluble, divalent state. Suitable reducing agents include, but are not limited to, hydroquinone and hydroxylated aromatic compounds, such as resorcinol, catechol, and the like. Suitable reducing agents are those disclosed in U.S. Patent No. 4,871,429. The amount of such reducing agent is well known to those skilled in the art, but is typically in the range of from about 0.1 g/L to about 5 g/L.

Bright deposits may be obtained by adding brighteners to the electrolyte compositions of the present invention. Such brighteners are well known to those skilled in the art. Suitable brighteners include, but are not limited to, aromatic aldehydes such as naphthaldehyde, benzaldehyde, allylbenzaldehyde, methoxybenzaldehyde and chlorobenzaldehyde, derivatives of aromatic aldehydes such as benzyl acetone and benzylidine acetone, aliphatic aldehydes such as acetaldehyde or glutaraldehyde, and acids such as acrylic acid, methacrylic acid and picolinic acid. Typically, brighteners are used at an amount of 0.1 to 3 g/L, and preferably 0.5 to 2 g/L.

It will be appreciated by those skilled in the art that hydroxy aromatic compounds or other wetting agents may be added to the electrolyte compositions of the present invention to provide further grain refinement. Such grain refiners may be added to the electrolyte composition of the present invention to further improve deposit appearance and operating current density range. Suitable other wetting agents include, but are not limited to: alkoxylates, such as the polyethoxylated amines JEFFAMINE T-403 or TRITON RW, or sulfated alkyl ethoxylates, such as TRITON QS-15, and gelatin or gelatin derivatives. The amounts of such grain refiners useful in the present invention are well known to those skilled in the art and typically are in the range of 0.01 to 20 mL/L, preferably 0.5 to 8 mL/L, and more preferably 1 to 5 mL/L.

Suitable non-ionic surfactants or wetting agents include, but are not limited to: relatively low molecular weight ethylene oxide ("EO") derivatives of aliphatic alcohols containing an alkyl group of up to 7 carbons or ethylene oxide derivatives of aromatic alcohols having up to two aromatic rings, which may be fused and which may be substituted with an alkyl group having up to 6 carbons. The aliphatic alcohols may be saturated or unsaturated. The aromatic alcohols typically have up to 20 carbon atoms prior to derivatization with ethylene oxide. Such aliphatic and aromatic alcohols may be further substituted, such as with sulfate or sulfonate groups. Suitable wetting agents include, but are not limited to: ethoxylated polystyrenated phenol containing 12 moles of EO, ethoxylated butanol containing 5 moles of EO, ethoxylated butanol containing 16 moles of EO, ethoxylated butanol containing 8 moles of EO, ethoxylated octanol containing 12 moles of EO, ethoxylated octylphenol containing 12 moles of EO, ethoxylated/propoxylated butanol, ethylene oxide/propylene oxide block copolymers, ethoxylated beta-naphthol containing 13 moles of EO, ethoxylated beta-naphthol containing 10 moles of EO, ethoxylated bisphenol A containing 10 moles of EO, ethoxylated bisphenol A containing 13 moles of EO, sulfated ethoxylated bisphenol A containing 30 moles of EO, and ethoxylated bisphenol A containing 8 moles of EO. Typically, such wetting agents are added in an amount of 0.1 to 20 g/L, and preferably 0.5 to 10 g/L.

Which optional additives, if any, are added to the electrolyte compositions of the present invention depends upon the results and types of deposits desired. It will be clear to one skilled in the art which additives and in what amounts are needed to achieve the desired finished deposit.

Conventional plating conditions may be used to electrolytically deposit tin or tin alloy layers on the nickel underlayers of the present invention. Typically, the tin or tin alloy plating bath is used at a temperature of from about 20° to about 60° C. Suitable current densities for tin or tin alloy electroplating are from 10 to 30 A/dm².

The present invention also provides a method of depositing a tin or tin alloy layer on a substrate including the steps of depositing a metal underlayer including nickel, nickel alloys, cobalt or cobalt alloys on a substrate; and then depositing a tin or tin alloy layer on the metal underlayer; wherein the metal underlayer has a thickness of up to 1 µm. A wide variety of substrates may be used in the present invention. Preferably, the substrate includes one or more metals that form an intermetallic compound with tin. Suitable substrates include, but are not limited to, copper or brass. It is preferred that the substrate includes a copper or copper alloy layer. Such copper alloys may contain small amounts of one or more other alloying elements.

Thus, the present invention further provides a substrate including a metal underlayer including nickel, nickel alloys, cobalt or cobalt alloys disposed on the substrate; and a tin or tin alloy layer disposed on the metal underlayer; wherein the metal underlayer has a thickness of up to 1 µm.

The present invention is particularly suitable for depositing a tin or tin alloy layer on an electronic device substrate. Suitable electronic device substrates include, but are not limited to, printed wiring board substrates, lead frames, semiconductor packages, components, connectors, contacts, chip capacitors, chip resistors and the like. Thus, the present invention still further provides an electronic device including a metal underlayer including nickel, nickel alloys, cobalt or cobalt alloys disposed on the device; and a tin or tin alloy layer disposed on the metal underlayer; wherein the metal underlayer has a thickness of up to 1 µm. Preferred electronic devices include those having an underlayer up to 0.75 µm. The present invention is particularly useful for plating lead frames. A particularly suitable tin alloy for use on such electronic devices is tin-copper, however other tin alloys may also be suitably used.

An advantage of the present invention is that whisker formation in tin and tin alloys is greatly reduced or even eliminated according to the present invention. A further advantage is that crack formation, particularly in the underlayer, is greatly reduced. Such reduced cracking provides electronic devices having reduced solderability problems.

The following examples are intended to illustrate further various aspects of the present invention, but are not intended to limit the scope of the invention in any aspect.

### Example 1

Samples were prepared by depositing a nickel underlayer on lead frames. The lead frames were commercially available copper lead frames including small amounts of alloying metals. The lead frames were either C 194 or C 151. The nickel layers were deposited using a commercially available nickel plating containing 11 g/L nickel chloride; 90 g/L of nickel as nickel sulfamate, 45 g/L boric acid and having a pII of about 4. The bath temperature was 55° C. The lead frames were immersed in the bath and the nickel layers were electrodeposited on the lead frames. The current density used was standard for the bath and lead frames plated. The lead frames were plated for a time sufficient to deposit the desired thickness of nickel. The samples were then removed from the nickel bath and rinsed and dried as required. The nickel layer thicknesses were determined by X-ray and are reported in Table 1.

**Table 1**

| Sample | Lead Frame Substrate | Nickel Layer Thickness (µm) |
|---|---|---|
| Control 1 | C 194 | 0 |
| Control 2 | C 151 | 0 |
| 1 | C 194 | 0.1 |
| 2 | " | 0.25 |
| 3 | " | 0.5 |
| 4 | " | 0.75 |
| 5 | " | 1 |
| Comparative 1 | " | 1.5 |
| Comparative 2 | " | 2 |
| 6 | C 151 | 0.1 |
| 7 | " | 0.25 |
| 8 | " | 0.5 |
| 9 | " | 0.75 |
| 10 | " | 1 |
| Comparative 3 | " | 1.5 |
| Comparative 4 | " | 2 |

### Example 2

The samples from Example 1 were electroplated with a tin-copper alloy using a plating bath containing 50 g/L tin as tin methanesulfonate, 3.6 g/L copper as copper methansulfonate, 130 mL/L methansulfonic acid, 4 g/L of ethoxylated non-ionic surfactant A, 0.75 g/L of ethoxylated/propoxylated non-ionic surfactant B, 4 g/L of complexing agent A, 1 g/L of complexing agent B, and 2 g/L of hydroquinone. The bath temperature was 40° C. The lead frame samples were plated at a current density of 15 A/dm² and were contacted with the tin-copper alloy bath for a time sufficient to deposit a 2 to 3 micron thick tin-copper alloy. The samples were evaluated by scanning electron microscopy for whisker growth after 6, 14 and 85 days. The results are reported in Table 2.

**Table 2**

| Sample | Whisker Growth After 6 Days | Whisker Growth After 14 Days | Whisker Growth After 85 Days |
|---|---|---|---|
| Control 1 | whisker | whisker | whisker |
| Control 2 | none | whisker | whisker |
| 1 | none | none | none |
| 2 | none | none | none |
| 3 | none | none | none |
| 4 | none | none | none |
| 5 | none | none | none |
| Comparative 1 | none | none | none |
| Comparative 2 | none | none | none |
| 6 | none | none | none |
| 7 | none | none | none |
| 8 | none | none | none |
| 9 | none | none | none |
| 10 | none | none | none |
| Comparative 3 | none | none | none |
| Comparative 4 | " | none | none |

The above data clearly show that very thin nickel layers reduce or eliminate the formation of tin whiskers. Further, the above data clearly show that very thin tin layers, i.e. tin layers of up to 1 µm, are as effective in reducing and/or eliminating tin whiskers as are conventional thick (≥ 2 µm) nickel layers.

## Claims

1. A method of reducing whisker formation comprising the steps of first depositing a metal underlayer comprising nickel, nickel alloys, cobalt or cobalt alloys; and then depositing a tin or tin alloy layer on the metal underlayer; wherein the metal underlayer has a thickness of up to 1 µm.

2. A method of depositing a tin or tin alloy layer on a substrate comprising the steps of depositing a metal underlayer comprising nickel, nickel alloys, cobalt or cobalt alloys on a substrate; and then depositing a tin or tin alloy layer on the metal underlayer; wherein the metal underlayer has a thickness of up to 1 µm.

3. The method of any one of claims 1 to 2 wherein the metal underlayer has a thickness of up to 0.8 µm.

4. The method of any one of claims 1 to 3 wherein the tin alloy comprises tin and one or more elements selected from lead, nickel, copper, bismuth, zinc, silver, antimony or indium.

5. The method of any one of claims 1 to 4 wherein the metal underlayer is selected from nickel, cobalt, nickel-cobalt, nickel-phosphorus or nickel-tungsten.

6. The method of any one of claims 1 to 5 wherein the tin or tin alloy is deposited electrolytically.

7. The method of any one of claims 1 to 6 wherein the tin or tin alloy layer has a thickness of 1 to 10 µm.

8. A substrate comprising a metal underlayer comprising nickel, nickel alloys, cobalt or cobalt alloys disposed on the substrate; and a tin or tin alloy layer disposed on the metal underlayer; wherein the metal underlayer has a thickness of up to 1 µm.

9. The substrate of claim 8 wherein the metal underlayer has a thickness of up to 0.8 µm.

10. A electronic device comprising a metal underlayer comprising nickel, nickel alloys, cobalt or cobalt alloys disposed on the device; and a tin or tin alloy layer disposed on the metal underlayer; wherein the metal underlayer has a thickness of up to 1 µm.
